# EUROPEAN PATENT APPLICATION

(11) **EP 4 461 203 A1**
(43) Date of publication of application: **13.11.2024**
(21) Application number: 23746717.0
(22) Date of filing: 16.01.2023
(51) Int. Cl.: A61B 1/045, A61B 1/313, A61B 34/20, G06T 7/55, G06T 7/70

(54) **MEDICAL INFORMATION PROCESSING SYSTEM, MEDICAL INFORMATION PROCESSING METHOD, AND PROGRAM**

(30) Priority: 31.01.2022 JP 2022012716
(71) Applicant: Sony Group Corporation, Tokyo 108-0075 (JP)
(72) Inventor: UYAMA Keisuke, Tokyo 108-0075 (JP)
(74) Representative: D Young & Co LLP
(86) International application number: PCT/JP2023/000946
(87) International publication number: WO 2023/145503

(57) **Abstract**

The present technology relates to a medical information processing system, a medical information processing method, and a program that enable a user to recognize reliability of additional information provided using three-dimensional information in a patient's body generated by SLAM or the like.

Three-dimensional information including a three-dimensional map of an operative field of a patient is generated, reliability of the three-dimensional information is determined, and a presentation form of additional information provided using the three-dimensional information is changed on the basis of the reliability.

## Description

### TECHNICAL FIELD

The present technology relates to a medical information processing system, a medical information processing method, and a program, and more particularly, to a medical information processing system, a medical information processing method, and a program that enable a user to recognize reliability of additional information provided using three-dimensional information in a patient's body generated by SLAM or the like.

### BACKGROUND ART

Patent Document 1 proposes a technology of generating three-dimensional information of an operative field on the basis of image information and sensor information in surgery using a medical observation device such as an endoscope and a microscope, and further providing information useful for surgery to an operator using the generated three-dimensional information.

### CITATION LIST

### PATENT DOCUMENT

Patent Document 1: Japanese Patent Application Laid-Open No. 2021-000258

### SUMMARY OF THE INVENTION

### PROBLEMS TO BE SOLVED BY THE INVENTION

In a method for generating three-dimensional information in a patient's body, such as simultaneous localization and mapping (SLAM), assuming a clinical site, the reliability of three-dimensional information changes according to the situation, and the reliability of additional information (annotation) provided using the generated three-dimensional information also changes. It is important for a user such as an operator to recognize how reliable the provided additional information is.

The present technology has been made in view of such a situation, and enables a user to recognize reliability of additional information provided using three-dimensional information in a patient's body generated by SLAM or the like.

### SOLUTIONS TO PROBLEMS

A medical information processing system or a program of the present technology is a medical information processing system including: a three-dimensional information generation unit that generates three-dimensional information including a three-dimensional map of an operative field of a patient; a reliability determination unit that determines reliability of the three-dimensional information; and a presentation control unit that changes a presentation form of additional information provided using the three-dimensional information on a basis of reliability determined by the reliability determination unit, or a program that causes a computer to function as such a medical information processing system.

A medical information processing method of the present technology is a medical information processing method of a medical information processing system including: a three-dimensional information generation unit; a reliability determination unit; and a presentation control unit, in which the three-dimensional information generation unit generates three-dimensional information including a three-dimensional map of an operative field of a patient, the reliability determination unit determines reliability of the three-dimensional information, and the presentation control unit changes a presentation form of additional information provided using the three-dimensional information on a basis of the reliability.

In the medical information processing system, the medical information processing method, and the program of the present technology, three-dimensional information including a three-dimensional map of an operative field of a patient is generated, reliability of the three-dimensional information is determined, and a presentation form of additional information provided using the three-dimensional information is changed on the basis of the reliability.

### BRIEF DESCRIPTION OF DRAWINGS

Fig. 1 is a diagram schematically illustrating an overall configuration of an operating room system.
Fig. 2 is a functional block diagram illustrating a functional configuration of a medical information processing system according to the present embodiment.
Fig. 3 is a diagram for describing a processing example of generating a three-dimensional map.
Fig. 4 is a functional block diagram obtained by adding a functional configuration related to setting of an annotation performed by an annotation two-dimensional position designation unit to Fig. 2.
Fig. 5 is a diagram illustrating a first setting form of annotation designation information.
Fig. 6 is a diagram illustrating a second setting form of annotation designation information.
Fig. 7 is a diagram illustrating the second setting form of annotation designation information.
Fig. 8 is a diagram illustrating the second setting form of annotation designation information.
Fig. 9 is a flowchart illustrating a procedure example of processing of setting an annotation.
Fig. 10 is a flowchart illustrating a procedure example of overall processing of displaying an annotation.
Fig. 11 is a diagram illustrating a state in which the density of a marking in an observation image displayed on an observation display unit is changed.
Fig. 12 is a flowchart illustrating a procedure example in a specific example of the processing of displaying an annotation.
Fig. 13 is a diagram illustrating a flow of processing of calculating the two-dimensional position of an annotation.
Fig. 14 is a flowchart illustrating a procedure example of the processing of calculating the two-dimensional position of an annotation.
Fig. 15 is a block diagram illustrating an example of a hardware configuration of an information processing device included in the medical information processing system according to an embodiment of the present technology.

### MODE FOR CARRYING OUT THE INVENTION

Hereinafter, an embodiment of the present technology will be described with reference to the drawings.

### <<Operating room system to which present technology is applied>>

The technology according to the present disclosure can be applied to various products. For example, the technology according to the present disclosure may be applied to an operating room system.

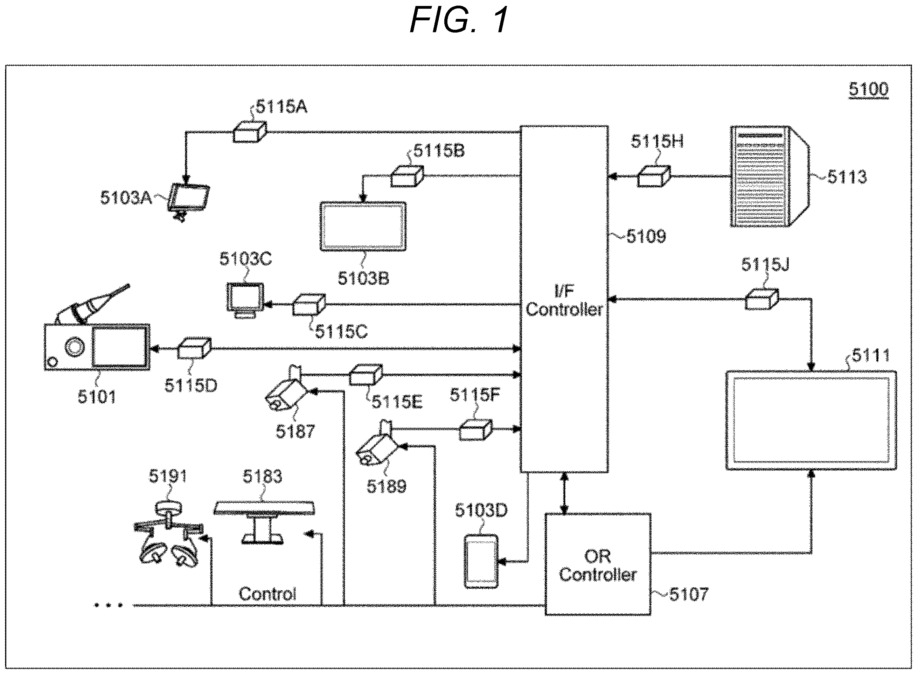

Fig. 1 is a diagram schematically illustrating an overall configuration of an operating room system 5100 to which the technology according to the present disclosure can be applied. Referring to Fig. 1, the operating room system 5100 is configured by connecting a group of devices installed in an operating room to be able to cooperate with each other via an operating room controller (OR Controller) 5107 and an input/output controller (I/F Controller) 5109. The operating room system 5100 is configured using an Internet Protocol (IP) network capable of transmitting and receiving 4K/8K images, and transmits and receives input and output images and control information for the devices via the IP network.

Various devices can be installed in the operating room. Fig. 1 illustrates, as an example, various groups of devices 5101 for endoscopic surgery, a ceiling camera 5187 that is provided on the ceiling of the operating room and captures an image of the hands of an operator, an operating field camera 5189 that is provided on the ceiling of the operating room and captures an image of the entire operating room, a plurality of display devices 5103A to 5103D, a patient bed 5183, and a light 5191. In addition to an endoscope illustrated in Fig. B1, various medical devices for acquiring images and videos, such as a master-slave endoscopic surgery robot and an X-ray imaging device, may be applied to the group of devices 5101.

The group of devices 5101, the ceiling camera 5187, the operating field camera 5189, and the display devices 5103A to 5103C are connected to the IF controller 5109 via IP converters 5115A to 5115F (hereinafter, denoted by reference numeral 5115 when not individually distinguished). The IP converters 5115D, 5115E, and 5115F on video source sides (camera sides) perform IP conversion on videos from individual medical image capturing devices (such as an endoscope, an operation microscope, an X-ray imaging device, an operating field camera, and a pathological image capturing device), and transmit the results on the network. The IP converters 5115A to 5115D on video output sides (monitor sides) convert the videos transmitted through the network into monitor-unique formats, and output the results. The IP converters on the video source sides function as encoders, and the IP converters on the video output sides function as decoders. The IP converters 5115 may have various image processing functions, and may have functions of, for example, resolution conversion processing corresponding to output destinations, rotation correction and image stabilization of an endoscopic video, and object recognition processing. The image processing functions may also include partial processing such as feature information extraction for analysis on a server described later. These image processing functions may be specific to the connected medical image devices, or may be upgradable from outside. The IP converters on the display sides can perform processing such as synthesis of a plurality of videos (for example, picture-in-picture (PinP) processing) and superimposition of annotation information. The protocol conversion function of each of the IP converters is a function to convert a received signal into a converted signal conforming to a communication protocol allowing the signal to be transmitted on the network (such as the Internet). Any communication protocol may be set as the communication protocol. The signal received by the IP converter and convertible in terms of protocol is a digital signal, and is, for example, a video signal or a pixel signal. The IP converter may be incorporated in a video source side device or in a video output side device.

The group of devices 5101 belong to, for example, an endoscopic surgery system, and include, for example, the endoscope and a display device for displaying an image captured by the endoscope. The display devices 5103A to 5103D, the patient bed 5183, and the light 5191 are, for example, devices equipped in the operating room separately from the endoscopic surgery system. Each of these devices for surgical or diagnostic is also called a medical device. The OR controller 5107 and/or the IF controller 5109 controls operations of the medical devices in cooperation. When the endoscopic surgery robot (surgery master-slave) system and the medical image acquisition devices such as an X-ray imaging device are included in the operating room, those devices can also be connected as the group of devices 5101 in the same manner.

The OR controller 5107 controls processing related to image display in the medical devices in an integrated manner. Specifically, the group of devices 5101, the ceiling camera 5187, and the operating field camera 5189 among the devices included in the operating room system 5100 can each be a device having a function to transmit (hereinafter, also called a transmission source device) information to be displayed (hereinafter, also called display information) during the operation. The display devices 5103A to 5103D can each be a device to output the display information (hereinafter, also called an output destination device). The OR controller 5107 has a function to control operations of the transmission source devices and the output destination devices so as to acquire the display information from the transmission source devices and transmit the display information to the output destination devices to cause the output destination devices to display or record the display information. The display information refers to, for example, various images captured during the operation and various types of information on the operation (for example, body information and past examination results of a patient and information about a surgical procedure).

Specifically, information about an image of a surgical site in a body cavity of the patient captured by the endoscope can be transmitted as the display information from the group of devices 5101 to the OR controller 5107. Information about an image of the area near the hands of the operator captured by the ceiling camera 5187 can be transmitted as the display information from the ceiling camera 5187. Information about an image representing the overall situation in the operating room captured by the operating field camera 5189 can be transmitted as the display information from the operating field camera 5189. When another device having an imaging function is present in the operating room system 5100, the OR controller 5107 may also acquire information about an image captured by the other device as the display information from the other device.

The OR controller 5107 displays the acquired display information (that is, the images captured during the operation and the various types of information on the operation) on at least one of the display devices 5103A to 5103D serving as the output destination devices. In the illustrated example, the display device 5103A is a display device installed on the ceiling of the operating room, being hung therefrom; the display device 5103B is a display device installed on a wall surface of the operating room; the display device 5103C is a display device installed on a desk in the operating room; and the display device 5103D is a mobile device (such as a tablet personal computer (PC)) having a display function.

The IF controller 5109 controls input and output of the video signal from and to connected devices. For example, the IF controller 5109 controls input and output of the video signal based on controlling of the OR controller 5107. The IF controller 5109 includes, for example, an IP switcher, and controls high-speed transfer of the image (video) signal between devices disposed on the IP network.

The operating room system 5100 may include a device outside the operating room. The device outside the operating room can be a server connected to a network built in and outside a hospital, a PC used by a medical staff, or a projector installed in a meeting room of the hospital. When such an external device is present outside the hospital, the OR controller 5107 can also display the display information on a display device of another hospital via, for example, a teleconference system for telemedicine.

An external server 5113 is, for example, an in-hospital server or a cloud server outside the operating room, and may be used for, for example, image analysis and/or data analysis. In this case, the video information in the operating room may be transmitted to the external server 5113, and the server may generate additional information through big data analysis or recognition/analysis processing using artificial intelligence (AI) (machine learning), and feed the additional information back to the display devices in the operating room. At this time, an IP converter 5115H connected to the video devices in the operating room transmits data to the external server 5113, so that the video is analyzed. The transmitted data may be, for example, a video itself of the operation using the endoscope or other tools, metadata extracted from the video, and/or data indicating an operating status of the connected devices.

The operating room system 5100 is further provided with a central operation panel 5111. Through the central operation panel 5111, a user can give the OR controller 5107 an instruction about input/output control of the IF controller 5109 and an instruction about an operation of the connected devices. Furthermore, the user can switch image display via the central operation panel 5111. The central operation panel 5111 is configured by providing a touchscreen on a display surface of a display device. The central operation panel 5111 may be connected to the IF controller 5109 via an IP converter 5115J.

The IP network may be established using a wired network, or a part or the whole of the network may be established using a wireless network. For example, each of the IP converters on the video source sides may have a wireless communication function, and may transmit the received image to an output side IP converter via a wireless communication network, such as the fifth-generation mobile communication system (5G) or the sixth-generation mobile communication system (6G).

### <Medical information processing system according to present embodiment>

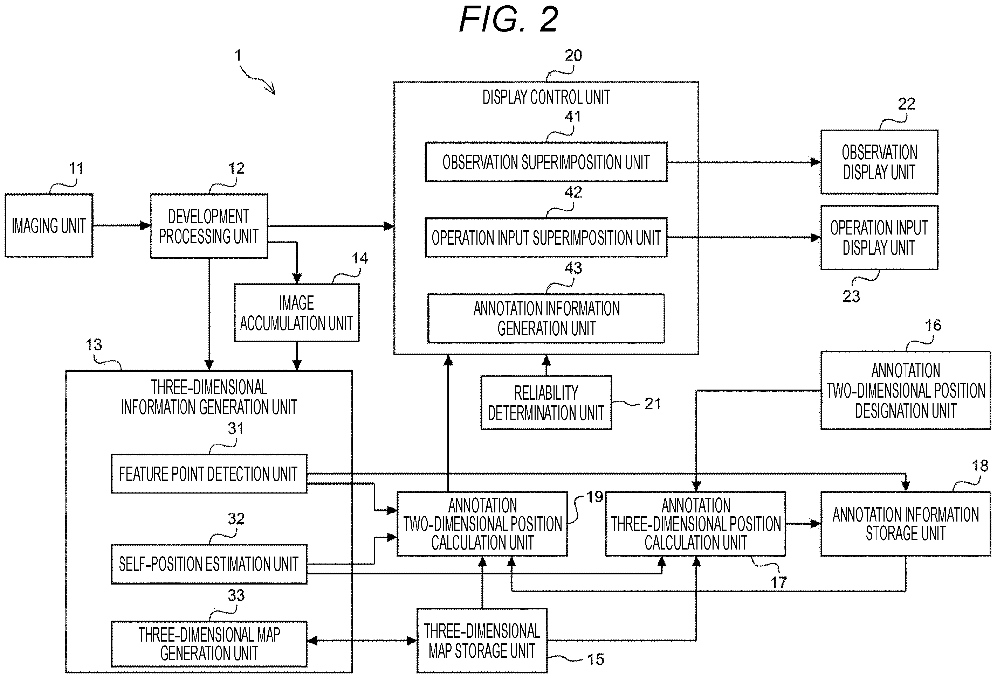

Next, a medical information processing system that forms a part of the operating room system 5100 will be described. Fig. 2 is a functional block diagram illustrating a functional configuration of a medical information processing system 1 according to the present embodiment. In Fig. 2, the medical information processing system 1 includes an imaging unit 11, a development processing unit 12, a three-dimensional information generation unit 13, an image accumulation unit 14, a three-dimensional map storage unit 15, an annotation two-dimensional position designation unit 16, an annotation three-dimensional position calculation unit 17, an annotation information storage unit 18, an annotation two-dimensional position calculation unit 19, a display control unit 20, a reliability determination unit 21, an observation display unit 22, and an operation input display unit 23.

### (Imaging unit 11)

The imaging unit 11 corresponds to an imaging device included at an endoscope distal end portion in an endoscopic surgery system to which the group of devices 5101 of Fig. 1 belongs. The imaging unit 11 captures an operative field in the body cavity of a patient, and supplies a captured image signal to the development processing unit 12. The imaging unit 11 includes a lens unit and an imaging element, and observation light having passed through the lens unit is condensed on a light receiving surface of the imaging element, and an image signal (RAW data) corresponding to an observation image is generated by photoelectric conversion. The imaging element is, for example, a complementary metal oxide semiconductor (CMOS) type image sensor.

Furthermore, the imaging unit 11 captures an observation image and a SLAM image (image for generating a three-dimensional map and image for self-position estimation). The observation image and the SLAM image may be the same image or may be different images. Each of the observation image and the SLAM image may be either a normal light image or a special light image. The normal light image is an image formed by irradiating an operative field with normal light (visible light) and forming the image with the normal light. The special light image is an image formed by irradiating an operative field with special light and forming the image with the special light. In a case of capturing the normal light image and the special light image, the imaging unit 11 may include individual imaging elements corresponding to the normal light image and the special light image, or may include only one imaging element capable of capturing both the normal light image and the special light image. Note that, in a case where the observation image and the SLAM image are captured by individual imaging elements, alignment processing of associating two-dimensional positions (coordinate values) of image points on the images corresponding to the same object point is performed. However, in order to simplify the description, in Fig. 2, a functional block for performing the alignment processing is omitted, and the observation image and the SLAM image are assumed to be images obtained by imaging the same angle of view range.

Here, the special light will be described.

### (1) In a case where IR light (infrared light) is used

As the special light, light such as IR light that enables observation of a deep blood vessel structure can be used. In a case where IR light is used for capturing the special light image as the SLAM image, it is possible to obtain a three-dimensional map that is not affected by a change in the state of the surface. That is, simultaneous localization and mapping (SLAM) that is hardly affected by treatment in surgery is implemented. Light having a wavelength longer than the wavelength of visible light, such as IR light, can be used as the special light. In this case, the wavelength band of the special light is a wavelength band longer than the wavelength band of visible light.

### (2) In a case where blue light is used

As the special light, blue light that enables observation of superficial blood vessels with the superficial blood vessels emphasized can be used. In a case where blue light is used for capturing the special light image as the SLAM image, feature points of SLAM are set in a blood vessel and the like that appear in the special light image. Since blood vessels are basically preserved in surgery, SLAM that is hardly affected by treatment in surgery is implemented.

### (3) In a case where highly transmissive light is used

As the special light, light that is more highly transmissive than visible light can be used. In a case where such special light is used, it is possible to obtain the special light image in which the operative field clearly appears even in a case where mist or fogging occurs in the organ. In a case where the special light image is used as the SLAM image, feature points can be obtained on the basis of the special light image, so that it is possible to reduce interruption of SLAM. Mist or the like occurs in the organ depending on the content of treatment, and in a case where a visible light image is used as the SLAM image, SLAM may not be able to be continued, but such a situation can be prevented.

### (4) In a case where polarized light is used as special light

Polarized light can be used as the special light. In a case where polarized light is used for capturing the special light image as the SLAM image, feature points in a specular reflection region can be obtained from the special light image, so that SLAM including the specular reflection region can be performed. During surgery, a region where specular reflection occurs may be formed in the operative field due to accumulation of water in the organ or the like. In a case where a visible light image is used as the SLAM image, feature points in the specular reflection region may not be able to be detected, but such a situation can be prevented by using the special light image. Polarized light to be the special light is generated using, for example, a polarizing filter. The visible light emitted from a light source passes through the polarizing filter so that polarized light is generated.

### (5) In a case where light that forms a known space pattern is used as special light

It is also possible to use light (structured light) that projects a known space pattern such as a checker pattern or a dot pattern as the special light. In a case where such special light is used for capturing the special light image as the SLAM image, it is possible to more accurately detect the three-dimensional shape of the operative field.

### (6) In a case where light to which pulse modulation is applied is used as special light

Light to which pulse modulation is applied can be used as the special light. In a case where such special light is used for capturing the special light image, it is possible to directly measure the distance to the operative field on the basis of the phase difference between reflected light and irradiation light. A three-dimensional map is generated on the basis of the measurement result of the distance to each position.

As described above, various types of light having a wavelength band different from the wavelength band of visible light can be used as the special light.

Note that the visible light is white light, and has, for example, a band of a predetermined wavelength with a lower limit in a range of approximately 360-400 nm and a predetermined wavelength with an upper limit in a range of approximately 760-830 nm. Various types of such light having a wavelength band different from the wavelength band of visible light can be used as the special light.

Instead of using light in a wavelength band different from that of visible light as the special light, light having a light source type (LED, laser, or the like) different from that of visible light may be used as the special light, or light having an irradiation intensity different from that of visible light may be used as the special light. It is also possible to use light with which a space pattern different from a space pattern irradiated with visible light is irradiated as the special light.

### (Development processing unit 12)

The development processing unit 12 performs development processing by applying a digital gain, a gamma curve, or the like to image signals (RAW data) of an observation image and a SLAM image from the imaging unit 11, thereby generating an observation image having excellent visibility and a SLAM image advantageous for SLAM processing such as feature point detection. The development processing unit 12 supplies generated observation images including a plurality of frames to the display control unit 132, and supplies generated SLAM images including a plurality of frames to the three-dimensional information generation unit 13.

### (Three-dimensional information generation unit 13)

The three-dimensional information generation unit 13 executes simultaneous localization and mapping (SLAM) on the basis of SLAM images from the development processing unit 12 and a three-dimensional map stored in the three-dimensional map storage unit 15. By the SLAM being generated, a three-dimensional map as three-dimensional information representing the shape of a space (observation space) including the operative field in the patient's body is generated, and self-position estimation at each timing is performed. The self-position estimation means to estimate the position and posture of a portion of the imaging unit 11 where an imaging element is disposed (for example, endoscope distal end portion). Hereinafter, the position and posture estimated by the self-position estimation are also referred to as the position/posture of the imaging unit 11. Note that the algorithm of the SLAM based on an image may be any algorithm that can generate a three-dimensional map or the self-position and posture on a three-dimensional map on the basis of feature points (feature amounts) in an image, and an existing algorithm may be adopted. For example, an algorithm such as PTAM, DTAM, ORB-SLAM, or Machine Learning SLAM that performs three-dimensional map and self-position estimation by machine learning using a multilayer neural network may be adopted. Furthermore, not only an image but also information of an inertial measurement unit (IMU) sensor or a depth sensor may be combined.

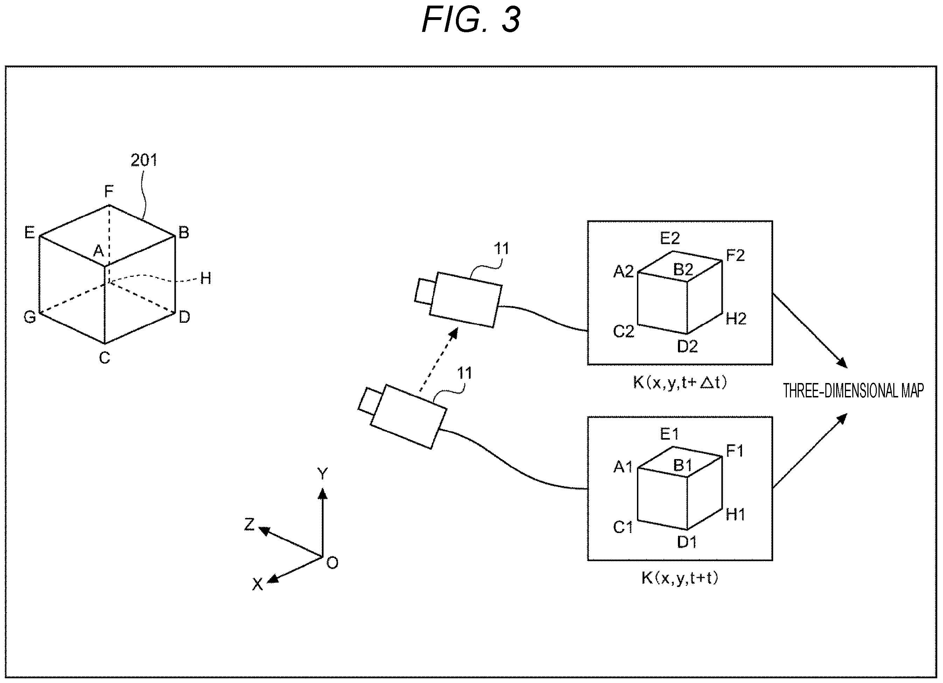

The three-dimensional information generation unit 13 includes a feature point detection unit 31, a self-position estimation unit 32, and a three-dimensional map generation unit 33. The feature point detection unit 31 detects feature points (and feature amounts) and two-dimensional coordinates (two-dimensional positions) of the feature points on SLAM images from the SLAM images from the development processing unit 12. The self-position estimation unit 32 and the three-dimensional map generation unit 33 estimate the position/posture of the imaging unit 11 using information of the feature points detected by the feature point detection unit 31 for at least two SLAM images obtained by capturing the operative field at different angles by the imaging unit 11, and generate (update as necessary) a three-dimensional map. A specific processing example of the three-dimensional information generation unit 13 will be described. Fig. 3 is a diagram for describing a processing example in which the three-dimensional information generation unit 13 generates a three-dimensional map.

Fig. 3 illustrates a state in which the imaging unit 11 observes a stationary object 201 in a three-dimensional space XYZ including a point on the space as a reference position O. Then, the imaging unit 11 captures (acquires) image data K(x,y,t) of a SLAM image at a time t, and captures image data K(x,y,t+Δt) of a SLAM image at a time t + Δt. Note that a time interval Δt is set to, for example, 33 msec or the like. Furthermore, the reference position O may be freely set, but for example, it is desirable to set the reference position O at a position that does not move with time. Note that x of the image data K(x,y,t) represents coordinates of the SLAM image in the horizontal direction, and y represents coordinates of the SLAM image in the vertical direction.

The feature point detection unit 31 detects feature points, which are pixels to be features, from the image data K(x,y,t) and the image data K(x,y,t+Δt). The feature points are, for example, pixels having pixel values different from that of the adjacent pixels by a predetermined value or more. Note that the feature points are desirably points that stably exist even after a lapse of time, and for example, pixels that constitute edges in an image are often used. Here, in order to simplify the following description, it is assumed that feature points A1, B1, C1, D1, E1, F1, and H1 that are vertices of the object 201 are detected from the image data K(x,y,t).

Next, the three-dimensional map generation unit 33 searches for points corresponding to the respective feature points A1, B1, C1, D1, E1, F1, and H1 from the image data K(x,y,t+Δt). Specifically, a point including a similar feature is searched from the image data K(x,y,t+Δt) on the basis of the pixel value of the feature point A1, a pixel value in the vicinity of the feature point A1, and the like (feature amount). Through this search processing, feature points A2, B2, C2, D2, E2, F2, and H2 corresponding to the feature points A1, B1, C1, D1, E1, F1, and H1 are detected from the image data K(x,y,t+Δt).

Subsequently, the three-dimensional map generation unit 33 calculates three-dimensional coordinates (XA,YA,ZA) of a point A on the space from, for example, two-dimensional coordinates of the feature point A1 on the image data K(x,y,t+Δt) and two-dimensional coordinates of the feature point A2 on the image data K(x,y,t+Δt) on the basis of the principle of three-dimensional surveying. In this way, the three-dimensional map generation unit 33 generates a three-dimensional map of the space in which the object 201 is placed as a set of the calculated three-dimensional coordinates (XA,YA,ZA). The three-dimensional map generation unit 33 stores information of the generated three-dimensional map in the three-dimensional map storage unit 15. Note that the three-dimensional map is an example of three-dimensional information in the present disclosure.

Furthermore, since the position/posture (position and posture) of an imaging device 2000 has changed during the time interval Δt, the self-position estimation unit 32 estimates the position/posture of the imaging unit 11. Mathematically, using the three-dimensional coordinates of each feature point that constitutes the object 201 and the position/posture of the imaging unit 11 as unknowns, simultaneous equations are created on the basis of the two-dimensional coordinates of the feature points observed in each of the image data K(x,y,t) and the image data K(x,y,t+Δt). The self-position estimation unit 32 solves the simultaneous equations to estimate the three-dimensional coordinates of each feature point that constitutes the object 201 and the position/posture of the imaging unit 11. As described above, by the feature points corresponding to the feature points detected from the image data K(x,y,t) and performing detection from the image data K(x,y,t+Δt) (that is, performing matching of feature points), the three-dimensional map generation unit 33 generates a three-dimensional map of the environment observed by the imaging unit 11. Moreover, the self-position estimation unit 32 can estimate the position/posture of the imaging unit 11, that is, the self-position. Furthermore, the three-dimensional map generation unit 33 can expand data of a three-dimensional map, for example, by making feature points that are not initially visible visible by repeatedly executing the above-described processing. Furthermore, since the three-dimensional map generation unit 33 repeatedly calculates the three-dimensional position of the same feature point by repeating the processing, the calculation error can be reduced by averaging processing being performed, for example. In this manner, the data of the three-dimensional map stored in the three-dimensional map storage unit 15 is updated as needed.

Information of the feature points detected by the feature point detection unit 31 (positions of the feature points, feature amounts, and the like in the SLAM images) is supplied to the annotation two-dimensional position calculation unit 19 and the annotation information storage unit 18 as necessary. The position/posture of the imaging unit 11 estimated by the self-position estimation unit 32 is supplied to the annotation two-dimensional position calculation unit 19 and the annotation three-dimensional position calculation unit 17 as necessary. Note that a technology for creating a three-dimensional map of the environment by matching of feature points and identifying the self-position of the imaging unit 11 is generally called simultaneous localization and mapping (SLAM) technology. The basic principle of the SLAM technology using a monocular camera is described, for example, in "Andrew J. Davison, "Real-Time Simultaneous Localization and Mapping with a Single Camera", Proceedings of the 9th IEEE International Conference on Computer Vision Volume 2, 2003, pp. 1403-1410". Furthermore, the SLAM technology for estimating the three-dimensional position of a subject using a camera image of the subject is also particularly referred to as Visual SLAM.

### (Image accumulation unit 14)

In Fig. 2, the image accumulation unit 14 stores SLAM images developed by the development processing unit 12 for a predetermined number of frames (for a certain period). As the SLAM images stored in the image accumulation unit 14, images captured later in time are prioritized. Therefore, in a case where the most recent SLAM image is generated by the development processing unit 12 after the SLAM images for the predetermined number of frames are accumulated in the image accumulation unit 14, the SLAM image stored at the oldest time among the SLAM images accumulated in the development processing unit 12 is deleted from the image accumulation unit 14, and the most recent SLAM image is stored in the image accumulation unit 14. The image accumulation unit 14 supplies the accumulated SLAM images to the three-dimensional information generation unit 13 as necessary (in a case where it is desired to quickly create a three-dimensional map, or the like).

### (Three-dimensional map storage unit 15)

The three-dimensional map storage unit 15 stores a three-dimensional map (three-dimensional map recognized from SLAM images) generated or updated by the three-dimensional map generation unit 33 of the three-dimensional information generation unit 13.

### (Annotation two-dimensional position designation unit 16)

The annotation two-dimensional position designation unit 16 designates the position of an annotation to be set on an observation image or a SLAM image for the annotation three-dimensional position calculation unit 17 as information for designating the position on a three-dimensional map of the annotation such as a marking to be superimposed on the observation image captured by the imaging unit 11. Note that, hereinafter, the position of the annotation on the three-dimensional map (three-dimensional coordinate system of the three-dimensional map) is also referred to as three-dimensional position of the annotation. The position of the annotation on the observation image or the SLAM image (two-dimensional coordinate system on the image) is also referred to as two-dimensional position of the annotation.

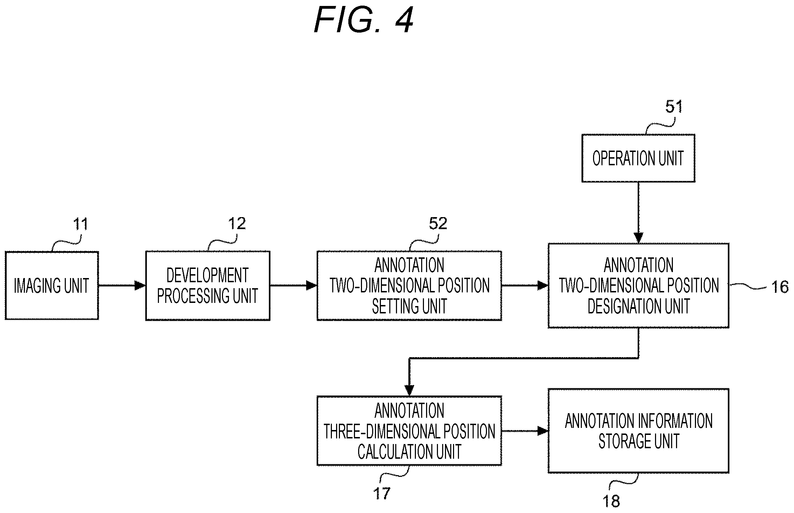

Here, the annotation two-dimensional position designation unit 16, the annotation three-dimensional position calculation unit 17, and the annotation information storage unit 18 in Fig. 2 will be described with reference to Fig. 4. Fig. 4 is a functional block diagram obtained by adding a functional configuration related to setting of an annotation performed by the annotation two-dimensional position designation unit 16 in Fig. 2 to Fig. 2. In the figure, portions corresponding to those of the medical information processing system 1 in Fig. 2 are denoted by the same reference signs.

In Fig. 4, in a case where a new annotation is set, information for designating the two-dimensional position of the annotation (referred to as annotation designation information) is set in the annotation two-dimensional position designation unit 16. Fig. 4 illustrates two forms of setting the annotation designation information together, and as a first setting form, the annotation designation information is set on the basis of operation information (operation input) indicating a user's operation from an operation unit 51. As a second setting form, the annotation designation information is set by an annotation two-dimensional position setting unit 52 on the basis of a SLAM image captured by the imaging unit 11, and the information is supplied to the annotation two-dimensional position designation unit 16.

### (First setting form of annotation designation information)

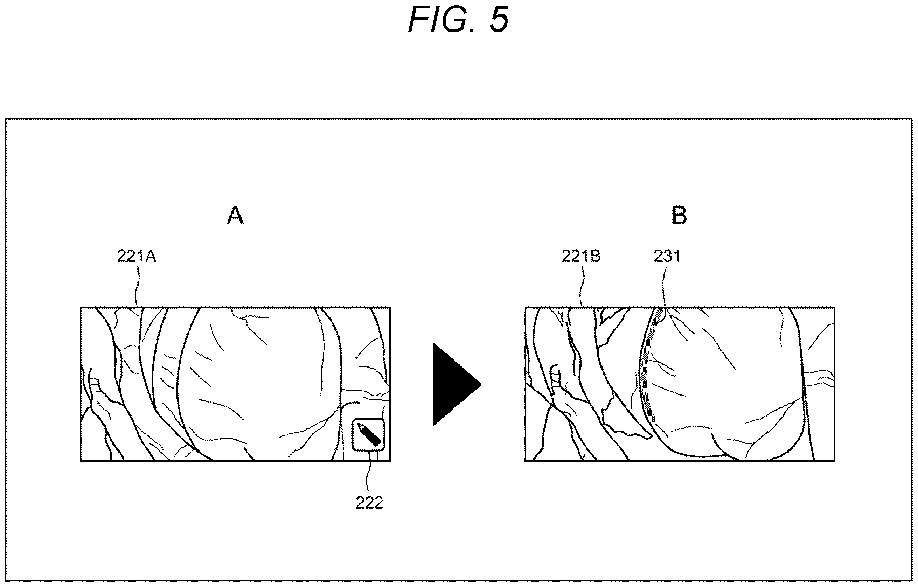

Describing the first setting form, the operation unit 51 is, for example, a touch panel installed on a display screen (also simply referred to as screen) of the operation input display unit 23 in Fig. 2. Note that the operation unit 51 may be any type of input device such as a pointing device such as a mouse or a keyboard. On the screen of the operation input display unit 23, as illustrated in A of Fig. 5, an observation image 221A of an operative field captured in real time by the imaging unit 11 (observation image on which an annotation is superimposed after setting of the annotation) is displayed. Note that a SLAM image may be displayed on the operation input display unit 23.

Furthermore, a writing mode button 222 is superimposed on the observation image 221A and displayed on the screen of the operation input display unit 23. In a case where a user (for example, medical advisor) performs a touch operation on the writing mode button 222, a writing mode is set, and the user can designate the position of an annotation by the touching operation (can set an annotation). Note that, in the writing mode, display of a real-time moving image and display of a still image may be switched by a predetermined operation. In the writing mode, in a case where the user performs a touch operation on a position on the screen (two-dimensional position on the observation image), the position on the observation image subjected to the touch operation by the user is supplied as operation information from the operation unit 51 to the annotation two-dimensional position designation unit 16. The position on the observation image subjected to the touch operation by the user is set in the annotation two-dimensional position designation unit 16 as annotation designation information for designating a position at which a marking as an annotation is to be set. For example, in the writing mode, it is possible to designate a position at which a marking such as a line is superimposed on the observation image by the user performing a touch operation such that swipe is performed on the screen. The annotation two-dimensional position designation unit 16 supplies the annotation designation information set in this manner to the annotation three-dimensional position calculation unit 17. In A of Fig. 5, in a case where the user performs a touch operation on the writing mode button 222 in the writing mode, the writing mode ends. In a case where the writing mode ends, the two-dimensional position of a marking designated by the user is associated as the position on the three-dimensional map (three-dimensional position) by processing of a functional block to be described below, and then, a marking 231 as the annotation designated by the user (medical advisor or the like) is superimposed on an observation image 221B of the operative field captured in real time by the imaging unit 11 as illustrated in B of Fig. 5 and displayed on the screen of the observation display unit 22 of Fig. 2 used by a user such as a surgeon. Also on the operation input display unit 23, an observation image similar to that in B of Fig. 5 on which a marking is superimposed is displayed. The position of the annotation is associated with the three-dimensional position on the three-dimensional map, and thus is also associated with the three-dimensional position in a real space. Therefore, in a case where the position/posture of the imaging unit 11 changes, the superimposition position of the annotation is changed in accordance with the position at which the three-dimensional position in the real space corresponding to the three-dimensional position of the annotation is reflected on the observation image 221B.

### (Second setting form of annotation designation information)

The second setting form will be described. In the second setting form, for example, it is particularly effective in a case where the imaging unit 11 captures a common normal light image as an observation image and a SLAM image and captures a special light image in addition to that, and such a case will be described as an example. The special light with which the operative field is irradiated so that the special light image is captured utilizes, for example, wavelength dependency of light absorption in a body tissue, and is light in a narrower band than irradiation light at the time of normal observation (that is, white light). Therefore, a special light image obtained by capturing a predetermined tissue such as a blood vessel in the mucosal surface layer with high contrast is captured. Alternatively, the special light may be excitation light for generating fluorescence from a body tissue, or may be excitation light corresponding to a fluorescence wavelength of a reagent such as indocyanine green (ICG) in a case where the reagent is locally injected into a body tissue.

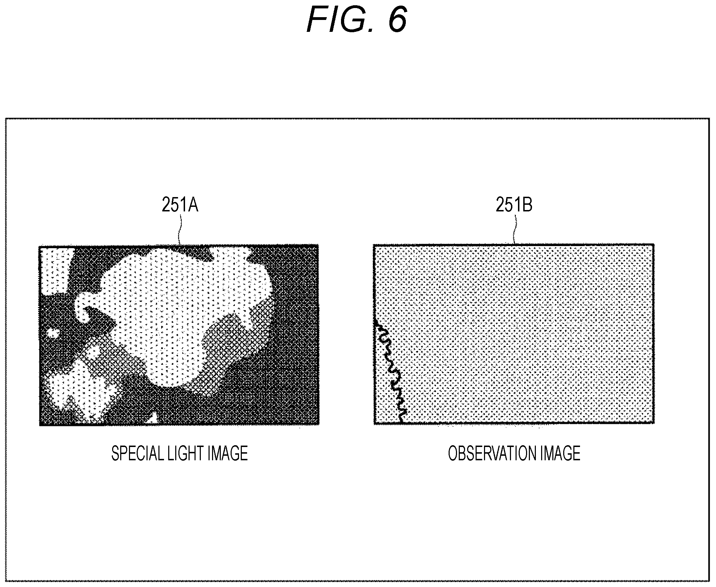

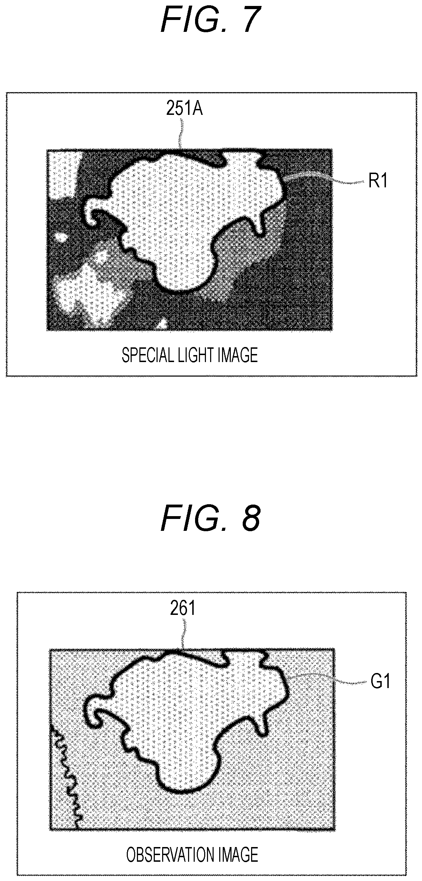

The annotation two-dimensional position setting unit 52 in Fig. 4 acquires a special light image captured by the imaging unit 11. Fig. 6 illustrates a special light image 251A captured by the imaging unit 11 and an observation image 251B that is a normal light image. As illustrated in Fig. 7, the annotation two-dimensional position setting unit 52 detects a region caused to fluoresce with a fluorescence intensity equal to or higher than a predetermined threshold in the special light image 251A as a region of interest R1. Note that the user may observe the special image and designate the region of interest R1, or the region of interest R1 may be detected using machine learning (inference model). The annotation two-dimensional position setting unit 52 supplies the two-dimensional position of the detected region of interest R1 on the special image to the annotation two-dimensional position designation unit 16. As a result, in the annotation two-dimensional position designation unit 16, the position on the special image of the region of interest R1 (corresponding to the position on the observation image) to which an annotation such as a marking is to be added is set as annotation designation information. The annotation two-dimensional position designation unit 16 supplies the annotation designation information set in this manner to the annotation three-dimensional position calculation unit 17. According to this, the two-dimensional position of the region of interest R1 to be the annotation is associated as the position on the three-dimensional map (three-dimensional position) by processing of a functional block to be described below, and then, an annotation G1 representing the region of interest R1 is superimposed on an observation image 261 of the operative field captured in real time by the imaging unit 11 as illustrated in Fig. 8 and displayed on the screen of the observation display unit 22 of Fig. 2 used by a user such as a surgeon. The position of the annotation G1 is associated with the three-dimensional position on the three-dimensional map, and thus is also associated with the three-dimensional position in a real space. Therefore, in a case where the position/posture of the imaging unit 11 changes, the superimposition position of the annotation G1 is changed in accordance with the position at which the three-dimensional position in the real space corresponding to the three-dimensional position of the annotation G1 is reflected on the observation image 261.

The first and second setting forms of annotation designation information described above differ only in how to designate an annotation to be set by the two-dimensional position, and are common after setting of the annotation designation information. The first and second setting forms of the annotation designation information are examples, and the present technology is not limited to a case where the designation by the two-dimensional position of an annotation to be set is performed by a specific method.

### (Annotation three-dimensional position calculation unit 17)

In Fig. 4, in a case where annotation designation information set in the first setting form, the second setting form, or the like is supplied from the annotation two-dimensional position designation unit 16, the annotation three-dimensional position calculation unit 17 acquires an estimation result of the position/posture of the imaging unit 11 and data of a three-dimensional map from the self-position estimation unit 32 and the three-dimensional map storage unit 15 in Fig. 2, respectively, and calculates the position on a three-dimensional map corresponding to the two-dimensional position of an annotation indicated by the annotation designation information as the three-dimensional position of the annotation on the basis of the acquired information. Specifically, the annotation three-dimensional position calculation unit 17 calculates which position on the three-dimensional map the two-dimensional position of the annotation indicated by the annotation designation information corresponds to on the basis of the current position/posture of the imaging unit 11.

### (Annotation information storage unit 18)

The annotation information storage unit 18 stores the three-dimensional position of an annotation (annotation information) calculated by the annotation three-dimensional position calculation unit 17. The annotation information stored in the annotation information storage unit 18 is not limited to a case of being calculated from the two-dimensional position of the annotation designated by annotation designation information of the annotation two-dimensional position designation unit 16. For example, the three-dimensional position corresponding to navigation information may be stored in the annotation information storage unit 18 as the annotation information on the basis of three-dimensional information of an organ generated in advance before surgery or the like using CT, MRI, or the like, or a three-dimensional position designated by the user or the like may be stored in the annotation information storage unit 18 as the annotation information on the basis of the three-dimensional information of the organ. The three-dimensional position of the annotation stored in the annotation information storage unit 18 may be set by various forms such that an object is simultaneously superimposed and displayed on an observation image, or the user may select which annotation is to be superimposed on the observation image.

Note that, in a case where the observation target in the body cavity moves or deforms, the three-dimensional position of the annotation stored in the annotation information storage unit 18 may be changed in accordance with the change. For example, in a case where the three-dimensional map generation unit 33 of the three-dimensional information generation unit 13 updates the three-dimensional map and the position of the region including the three-dimensional position of the annotation has changed, a change of the three-dimensional position of the annotation is performed in addition to update of the three-dimensional map. Moreover, the reliability of the entire three-dimensional map may be improved by adding time information indicating the time of generation or update to data of the three-dimensional map of each position. By updating the three-dimensional map such that the most recent data having high reliability is reserved, it is possible to follow and display an annotation even if movement or deformation occurs in the observation target in the body cavity.

### (Annotation two-dimensional position calculation unit 19)

In Fig. 2, the annotation two-dimensional position calculation unit 19 calculates the two-dimensional position of an annotation to be superimposed on an observation image on the basis of a three-dimensional map stored in the three-dimensional map storage unit 15, annotation information stored in the annotation information storage unit 18, and the position/posture of the imaging unit 11 estimated by the self-position estimation unit 32 of the three-dimensional information generation unit 13. That is, the annotation two-dimensional position calculation unit 19 calculates which position on an observation image the three-dimensional position of the annotation corresponds to on the basis of the three-dimensional map, the position/posture of the imaging unit 11, and the three-dimensional position of the annotation. The annotation two-dimensional position calculation unit 19 supplies the calculated two-dimensional position of the annotation to the display control unit 20.

In a case where occurrence of some error is detected, such as a case where the self-position estimation unit 32 of the three-dimensional information generation unit 13 has failed to estimate the position/posture of the imaging unit 11, the annotation two-dimensional position calculation unit 19 calculates the two-dimensional position of an annotation using information such as feature points on a SLAM image (annotation feature point information) instead of calculating the two-dimensional position of the annotation from information of the three-dimensional position of the annotation. Therefore, the annotation information storage unit 18 stores, as annotation feature point information as a part of annotation information, information of feature points on a SLAM image (feature point information including the two-dimensional positions of the feature points, the feature amounts, and the like) detected by the feature point detection unit 31 of the three-dimensional information generation unit 13 in a case where the annotation designation information (two-dimensional position of an annotation on an observation image) is set to the annotation two-dimensional position designation unit 16 in Fig. 4 and information indicating positional relationships between the two-dimensional position of the annotation and the feature points (positional relationship information). However, the annotation information storage unit 18 may store annotation feature point information detected in, instead of a case where the annotation designation information is set in the annotation two-dimensional position designation unit 16 in Fig. 4, a case where the self-position estimation unit 32 of the three-dimensional information generation unit 13 normally estimates the position/posture of the imaging unit 11.

### (Display control unit 20)

The display control unit 20 controls display of an observation image supplied from the development processing unit 12 on the observation display unit 22 and the operation input display unit 23. The display control unit 132 includes an observation superimposition unit 41, an operation input superimposition unit 42, and an annotation information generation unit 43. The observation superimposition unit 41 generates an observation image obtained by superimposing an annotation on an observation image from the development processing unit 12 and supplies the observation image to the observation display unit 22. The operation input superimposition unit 42 generates an observation image obtained by superimposing an annotation on an observation image from the development processing unit 12 and supplies the observation image to the operation input display unit 23. The observation image supplied to the observation display unit 22 and the observation image supplied to the operation input display unit 23 are different from each other in whether or not a user interface image with which the user performs an operation input is superimposed as the writing mode button 222 in the observation image 221A in A of Fig. 5, and other points are substantially common.

The observation superimposition unit 41 and the operation input superimposition unit 42 superimpose an image of the annotation stored in the annotation information storage unit 18 on the two-dimensional position of the annotation supplied from the annotation two-dimensional position calculation unit 19. Examples of the image of the annotation stored in the annotation information storage unit 18 include an image of the marking 231 as an annotation designated by the user as illustrated in B of Fig. 5, an image of the annotation G1 indicating a region of interest as illustrated in Fig. 8, and the like.

The annotation information generation unit 43 generates an image of an annotation to be superimposed on an observation image, supplies the image to the observation superimposition unit 41 and the operation input superimposition unit 42, and causes the observation superimposition unit 41 and the operation input superimposition unit 42 to superimpose the image on the observation image. The image of the annotation to be superimposed on the observation image is changed to a form according to a determination result (reliability to be described below) of the reliability determination unit 21 in Fig. 2. For example, the form of the density (transparency), color, thickness (in a case of a line image), and display state (blinking speed or the like in a case of blinking display) of the image of the annotation to be superimposed on the observation image is changed according to the reliability. Furthermore, the annotation information generation unit 43 generates not only the image of the annotation stored in the annotation information storage unit 18 but also an image of an annotation such as a character, a symbol, or a figure to be superimposed on a predetermined position on an observation image on the basis of a determination result (reliability) of the reliability determination unit 21 or the like, and supplies the generated image to the observation superimposition unit 41 or the operation input superimposition unit 42.

### (Reliability determination unit 21)

The reliability determination unit 21 determines (evaluates) the reliability on the basis of information related to the reliability of the position of an annotation to be superimposed on an observation image, and supplies a determination result (value indicating the reliability) to the display control unit 20. The reliability may be evaluated on the basis of one or more pieces of information, and a plurality of types of reliability can be evaluated. The reliability is, for example, evaluated in stages by a plurality of level values and the higher the level value, the higher the reliability. However, the representation of the reliability is not limited thereto.

The reliability of the position of an annotation to be superimposed on an observation image can be evaluated by the reliability of a three-dimensional map indicating the degree of coincidence of the three-dimensional map generated by the three-dimensional map generation unit 33 with the actual space and the reliability of self-position estimation indicating the degree of coincidence of the position/posture of the imaging unit 11 estimated by the self-position estimation unit 32 with the actual position/posture. Since the reliability of the three-dimensional map and the reliability of the self-position estimation affect each other, the reliability representing at least one of the reliability of the three-dimensional map or the reliability of the self-position estimation is referred to as the reliability of the three-dimensional map and the self-position estimation or simply the reliability. The reliability can be, for example, quantitatively evaluated on the basis of the following first to fourth information (reliability evaluation information).

The first reliability evaluation information is an elapsed time from a time point at which data of a three-dimensional map around a region corresponding to a three-dimensional position of an annotation is generated or a time point at which the three-dimensional position of the annotation is set. The first reliability evaluation information is due to a high possibility that the situation of the actual space (operative field) changes with the lapse of time.

The second reliability evaluation information is the data amount of a three-dimensional map generated by the three-dimensional map generation unit 33 (number of coordinate points (map points) at which an object exists), in particular, the data amount of a three-dimensional map of a region corresponding to the three-dimensional position at which an annotation is set. The second reliability evaluation information is due to a high possibility that the detailed shape of the actual space cannot be recognized in a case where the data amount of a three-dimensional map is small.

The third reliability evaluation information is information regarding feature points detected for SLAM images by the feature point detection unit 31. The information regarding feature points includes, for example, the number of feature points detected from SLAM images, the number of key frames in which the feature points are detected in a certain period of time, the magnitude of a difference in viewpoints of the key frames in which the feature points are detected in a certain period of time, and the like. The key frames represent frames in which detected feature points are reflected in a three-dimensional map among individual frames of continuously captured SLAM images. The third reliability evaluation information indicates a possibility that matching and tracking of feature points are not appropriately performed or a possibility that the shape of the actual space (operative field) has greatly changed. Therefore, the third reliability evaluation information is due to capability of evaluating a possibility that the estimated position/posture of the imaging unit 11 is different from the actual position/posture or a possibility that a three-dimensional map is different from the actual space.

The fourth reliability evaluation information is the presence or absence of an estimation result of the position/posture of the imaging unit 11 by the self-position estimation unit 32 (whether or not estimation has been performed). The fourth reliability evaluation information is due to the fact that the position/posture of the imaging unit 11 cannot be correctly recognized.

The reliability determination unit 21 acquires any one or a plurality of pieces of reliability evaluation information among a plurality of types of reliability evaluation information as the first to fourth, quantitatively determines (evaluates) the reliability on the basis of the reliability evaluation information for each, and calculates a level value indicating one or a plurality of types of reliability as a determination result. The reliability as a determination result in the reliability determination unit 21 is appropriately referred to for each of various types of processing, and processing according to the reliability is executed.

### (Observation display unit 22)

The observation display unit 22 in Fig. 2 corresponds to the endoscope of the endoscopic surgical system to which the group of devices 5101 in Fig. 1 belongs, a display device that displays an image captured by the endoscope, or any of the display devices 5103A to 5103D. The observation display unit 22 displays an observation image generated by the observation superimposition unit 41 of the display control unit 20 for the main purpose of presenting information to the user by the observation image captured by the imaging unit 11 and an annotation superimposed on the observation image.

### (Operation input display unit 23)

The operation input display unit 23 corresponds to the endoscope of the endoscopic surgical system to which the group of devices 5101 in Fig. 1 belongs, a display device that displays an image captured by the endoscope, or any of the display devices 5103A to 5103D. The operation input display unit 23 displays an observation image generated by the operation input superimposition unit 42 of the display control unit 20 for the purpose of presenting information to the user by the observation image captured by the imaging unit 11 and an annotation superimposed on the observation image, and operation input by the user of the annotation superimposed on the observation image.

Components other than the imaging unit 11 of the medical information processing system 1 illustrated in Figs. 2 and 4 described above can be constructed by a program executed in the external server 5113 of the operating room system 5100 in Fig. 1. Furthermore, some or all of the components other than the imaging unit 11 of the medical information processing system 1 can be constructed in any of the OR controller 5107, the IF controller 5109, and the IP converters 5115A to 5115F, and the components other than the imaging unit 11 of the medical information processing system 1 can be constructed in a distributed manner in a plurality of components of the operating room system 5100 in Fig. 1.

### <<Processing according to reliability>>

Hereinafter, processing according to the reliability of a three-dimensional map and self-position estimation that is a determination result of the reliability determination unit 21 will be described.

### <Procedure example of processing of setting annotation>

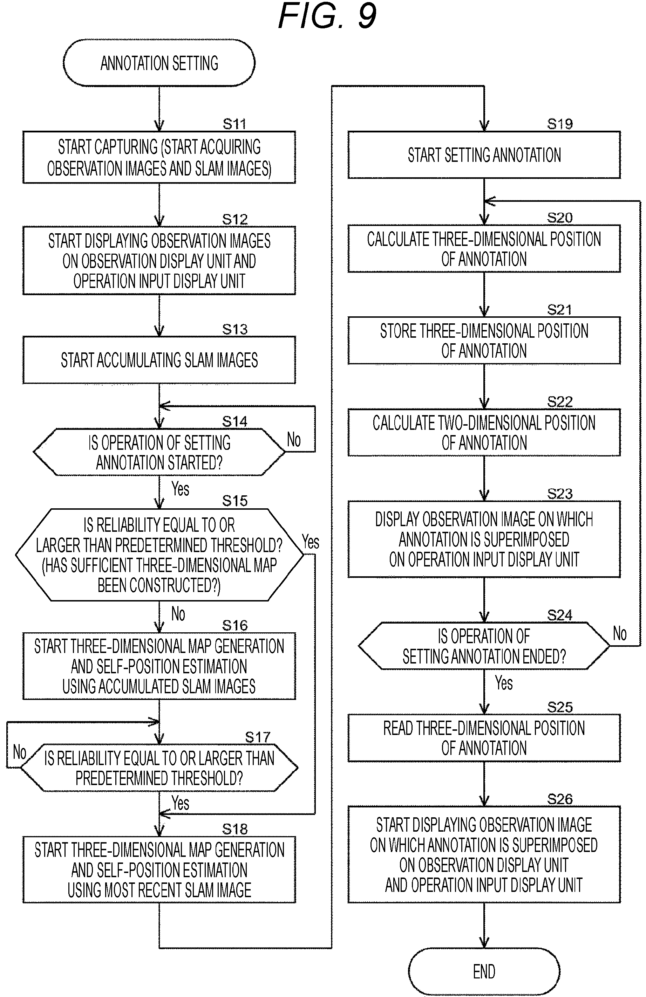

Fig. 9 is a flowchart illustrating a procedure example of processing of setting an annotation to be superimposed on observation images by the medical information processing system 1 of Fig. 2. In step S11, the imaging unit 11 starts capturing observation images and SLAM images. Thereafter, the imaging unit 11 repeatedly captures the observation images and the SLAM images at a predetermined period. The processing proceeds from step S11 to step S12. In step S12, the display control unit 20 (observation superimposition unit 41 and operation input superimposition unit 42) starts displaying the observation images captured by the imaging unit 11 on the observation display unit 22 and the operation input display unit 23. The processing proceeds from step S12 to step S13. In step S13, the image accumulation unit 14 starts accumulating the SLAM images captured by the imaging unit 11. Note that, in a case where the image accumulation unit 14 accumulates the SLAM images for a predetermined number of frames, the SLAM image stored at the oldest time is deleted from the image accumulation unit 14, and the most recent SLAM image is stored in the image accumulation unit 14. The processing proceeds from step S13 to step S14.

In step S14, the annotation two-dimensional position designation unit 16 determines whether or not an operation of setting an annotation is executed (started). For example, in the first setting form of annotation designation information described with reference to Fig. 5, it is determined whether or not a touch operation has been performed on the writing mode button 222 displayed on the screen of the operation input display unit 23.

In a case where the determination is negative in step S14, the processing repeats step S14, and in a case where the determination is affirmative, the processing proceeds to step S15. In step S15, the reliability determination unit 21 determines whether or not the reliability of a three-dimensional map and self-position estimation is equal to or larger than a predetermined threshold with reference to the three-dimensional map storage unit 15. In this processing, it is assumed that the reliability based on the above-described second reliability evaluation information is used as the reliability, and for example, the larger the data amount of the three-dimensional map (number of coordinate points on which an object exists) is, the higher the level value is set. That is, the determination processing in step S15 corresponds to determining whether or not a sufficient three-dimensional map has been constructed. Note that, instead of the data amount of the entire three-dimensional map, the data amount of the three-dimensional map that is limited to a region of the three-dimensional map corresponding to the position of a real space captured at the position and posture of the imaging unit 11 estimated by the self-position estimation unit 32 of the three-dimensional information generation unit 13 may be referred to. In a case where setting of an annotation is performed for the first time, a negative result is obtained in the determination in step S15 since the three-dimensional map is not generated, the reliability of the three-dimensional map is 0, which is the lowest level value, and a threshold is set to at least a value larger than 0.

In a case where the determination is affirmative in step S15, the processing proceeds to step S18, and in a case where the determination is negative, the processing proceeds to step S16. In step S16, the three-dimensional information generation unit 13 acquires the past SLAM images accumulated in the image accumulation unit 14, and starts three-dimensional map generation processing of generating a three-dimensional map and self-position estimation processing of estimating the self-position (position/posture of the imaging unit 11) on the basis of the acquired SLAM images. At this time, among the SLAM images accumulated in the image accumulation unit 14, the three-dimensional information generation unit 13 may acquire the images in order from one with a later (newer) imaging time (accumulation time) or in order from one with an earlier (older) imaging time. The processing proceeds from step S16 to step S17. In step S17, as in step S15, the reliability determination unit 21 determines whether or not the reliability of the three-dimensional map is equal to or larger than a predetermined threshold. Note that the data amount of the three-dimensional map gradually increases with the start of generation of the three-dimensional map in step S16, and the reliability also increases. In a case where the determination is negative in step S17, the processing repeats step S17, and in a case where the determination is affirmative, the processing proceeds to step S18.

In step S18, the three-dimensional information generation unit 13 starts three-dimensional map generation processing and self-position estimation processing using the most recent SLAM image captured by the imaging unit 11. The processing proceeds from step S18 to step S19. In step S19, the annotation two-dimensional position designation unit 16 starts setting an annotation (annotation designation information). For example, in a case where setting of an annotation is performed in the form described with reference to Fig. 5 as the first setting form of annotation designation information, the annotation two-dimensional position designation unit 16 starts processing of accepting an operation by the user of writing a marking as an annotation in an observation image. The processing proceeds from step S19 to step S20. In step S20, the annotation three-dimensional position calculation unit 17 calculates the three-dimensional position (position on the three-dimensional map) of the annotation on the basis of annotation designation information set or in the process of being set in the annotation two-dimensional position designation unit, the three-dimensional map of the three-dimensional map storage unit 15, and the position/posture of the imaging unit 11 estimated by the self-position estimation unit 32 of the three-dimensional information generation unit 13. The processing proceeds from step S20 to step S21. In step S21, the annotation three-dimensional position calculation unit 17 stores the three-dimensional position of the annotation calculated in step S20 in the annotation information storage unit 18. The processing proceeds from step S21 to step S22. In step S22, the annotation two-dimensional position calculation unit 19 calculates the position of the annotation on the observation image (two-dimensional position of the annotation) on the basis of the three-dimensional position of the annotation stored in the annotation information storage unit 18, the three-dimensional map stored in the three-dimensional map storage unit 15, and the position/posture of the imaging unit 11 estimated by the self-position estimation unit 32 of the three-dimensional information generation unit 13. The processing proceeds from step S22 to step S23.

In step S23, the display control unit 20 (operation input superimposition unit 42) superimposes the annotation on the two-dimensional position of the annotation calculated in step S22 on the observation image from the development processing unit 12, and causes the operation input display unit 23 to display the observation image on which the annotation is superimposed. Furthermore, the display control unit 20 superimposes notification information (characters or the like) notifying that the position of the annotation is displayed according to the three-dimensional map generated using the SLAM images captured in the past on the image for observation as an annotation. The processing proceeds from step S23 to step S24.

In step S24, the annotation two-dimensional position designation unit 16 determines whether or not the operation of setting an annotation is ended. For example, in the first setting form of annotation designation information described with reference to Fig. 5, it is determined whether or not a touch operation has been performed on the writing mode button 222 displayed on the screen of the operation input display unit 23. In a case where the determination is negative in step S24, the processing returns to step S20, and step S20 to step S24 are repeated. In a case where the determination is affirmative in step S24, the processing proceeds to step S25. In step S25, the annotation two-dimensional position calculation unit 19 reads the three-dimensional position of the annotation stored in the annotation information storage unit 18 in step S21. The processing proceeds to step S26. In step S26, the display control unit 20 (operation input superimposition unit 42) starts displaying the observation image on which the annotation is superimposed on the two-dimensional position of the annotation obtained by calculation that is similar to step S22 and omitted in the flowchart on the observation display unit 22 and the operation input display unit 23.

Thus, the processing of setting an annotation ends. However, after the processing of setting an annotation is completed, the display of the observation image on which the annotation is superimposed in step S26 on the observation display unit 22 and the operation input display unit 23 is continuously performed, and the processing from step S14 is repeated. In a case where it is determined in step S14 that the second and subsequent setting of an annotation is started, and a sufficient three-dimensional map is constructed in step S15, the three-dimensional information generation unit 13 performs three-dimensional map generation processing and self-position estimation processing on the basis of the most recent SLAM image captured by the imaging unit 11 without using the SLAM images accumulated in the image accumulation unit 14.

According to the processing of setting an annotation described above, a waiting time and effort after the user or the like performs an operation to set an annotation such as a marking are reduced. Conventionally, initialization and construction of a three-dimensional map are started after the user performs an operation to set an annotation, and thus a waiting time until the setting of the annotation becomes possible is long. Furthermore, in order to construct a three-dimensional map, it is necessary to intentionally change the position/posture of the imaging unit 11, which takes a time and effort.

On the other hand, in the processing of setting an annotation in Fig. 9, a three-dimensional map is constructed at high speed using automatically accumulated SLAM images (a frame can be skipped because tracking is not required) before the user or the like performs an operation to set an annotation such as a marking, so that the setting of an annotation can be started with an extremely short waiting time. Furthermore, since an annotation can be set in a state where a three-dimensional map is sufficiently constructed, the quality (reliability) of the annotation such as a marking is high. Note that the three-dimensional map may be generated using the SLAM images accumulated in the image accumulation unit 14 at certain time intervals without waiting for a user's operation.

### <Procedure example of processing of displaying annotation according to reliability>

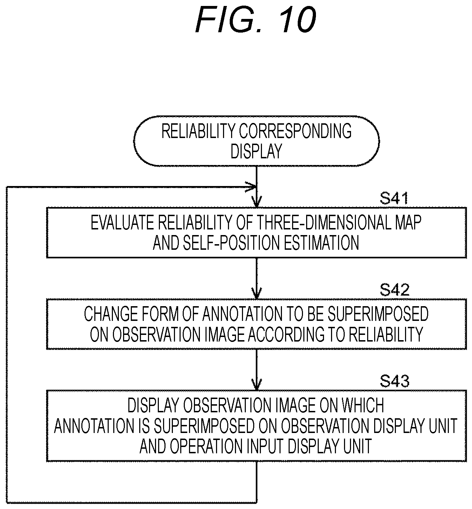

Fig. 10 is a flowchart illustrating a procedure example of overall processing of displaying an annotation. Note that it is assumed that capturing of an observation image and a SLAM image by the imaging unit 11 and display of the observation image on which an annotation is superimposed on the observation display unit 22 and the operation input display unit 23 have been performed.

In step S41, the reliability determination unit 21 evaluates the reliability of a three-dimensional map and self-position estimation, and calculates a level value indicating the reliability. Here, the type of the reliability to be evaluated is not limited to a specific type, and is appropriately selected according to the type of the reliability to be in notification to the user (to be recognized by the user). The processing proceeds from step S41 to step S42.

In step S42, the annotation information generation unit 43 of the display control unit 20 sets or changes the form of the annotation to be superimposed on the observation image according to the reliability (level value) calculated in step S41. For example, the form of the density (transparency), color, thickness (in a case of a line image), display state, and display state (blinking speed or the like in a case of blinking display) of the image of the annotation is changed. Furthermore, the annotation information generation unit 43 sets or changes the image of the annotation such as a character, a symbol, or a figure to be superimposed at a predetermined position on the observation image on the basis of a determination result (reliability) of the reliability determination unit 21 or the like. The processing proceeds from step S42 to step S43.

In step S43, the observation superimposition unit 41 and the operation input superimposition unit 42 of the display control unit 20 superimpose the annotation set or changed in step S42 on the observation image from the development processing unit 12 and display the same on the observation display unit 22 and the operation input display unit 23, respectively.

By the above processing procedure, the form of the annotation to be superimposed on the observation image is changed according to the reliability. According to the processing of displaying an annotation in Fig. 10, the user can visually recognize the degree of reliability of the position of an annotation to be superimposed on an observation image. The notification of the reliability to the user is not limited to the case of display on the observation image, and may be a case of using a physical means (light, vibration, or the like) by any device.

### <Specific example of processing of displaying annotation according to reliability>

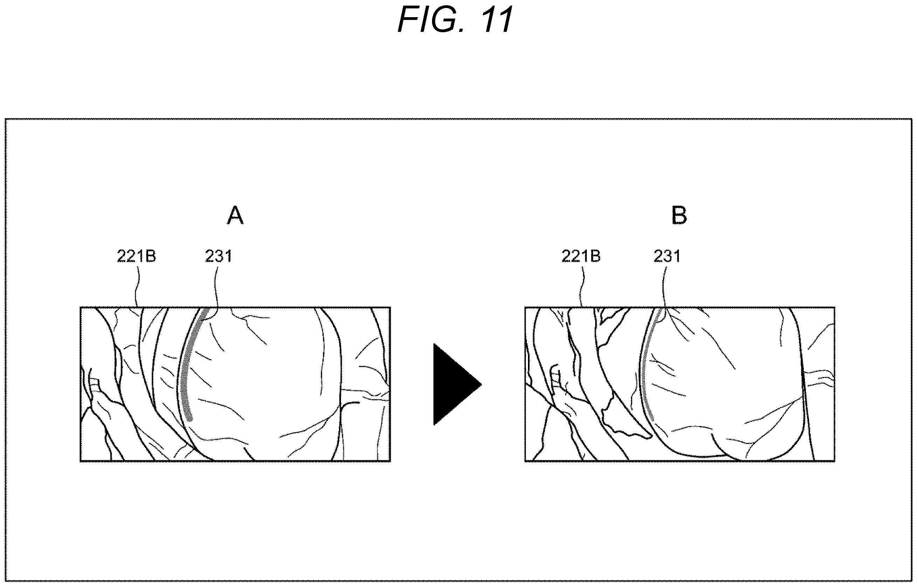

A case where the processing of displaying an annotation in Fig. 10 is applied to display of the annotation (marking 231) in Fig. 5 (B of Fig. 5) used in the description of the first setting form of annotation designation information will be described. In Fig. 5, according to the first setting form of annotation designation information, the marking 231 as the annotation designated by the user (medical advisor or the like) is superimposed and displayed on the observation image 221B of the operative field captured in real time by the imaging unit 11. In the present specific example, the form of the annotation is changed according to the reliability evaluated on the basis of the above-described first reliability evaluation information. It is assumed that the first reliability evaluation information is an elapsed time from a time point at which the three-dimensional position of the annotation is set. In a case where the three-dimensional position of the annotation based on user operation information is set by the annotation three-dimensional position calculation unit 17 in Fig. 2, the reliability determination unit 21 measures the elapsed time from that time. Assuming that the reliability is evaluated in six stages of 0 to 5, the reliability determination unit 21 evaluates the reliability as 5 at a time point at which the measurement of the elapsed time is started (time point at which the measurement time is 0). Then, every time the measurement time elapses for a certain period of time, the reliability is decreased from 5 by 1. The annotation information generation unit 43 of the display control unit 20 acquires the value (level value) of the reliability evaluated by the reliability determination unit 21. In a case of generating the image of the marking 231 as the annotation illustrated in B of Fig. 5 to be superimposed on the observation image, the annotation information generation unit 43 maximizes the density of the marking 231 in a case where the reliability is 5 and minimizes the density in a case where the reliability is 0, and decreases the density of the marking 231 as the reliability decreases. Similarly to B of Fig. 5, Fig. 11 is a diagram illustrating a state in which the density of the marking 231 in the observation image 221B displayed on the observation display unit 22 is changed. A of Fig. 11 is the same as B of Fig. 5, and represents the observation image 221B at a time point at which the marking 231 is set. On the other hand, B of Fig. 11 illustrates the observation image 221B in a case where the reliability decreases after a lapse of a certain period of time from the time point at which the marking 231 is set. According to this, in a case where the reliability decreases, the density of the image of the marking 231 decreases as illustrated in B of Fig. 11, and the user can visually easily recognize that the reliability of the position of the marking 231 decreases. Note that the reliability may be determined on the basis of any of the above-described first to fourth reliability evaluation information, for example, and may be particularly determined on the basis of the third reliability evaluation information. Furthermore, detection of a large change in scene due to excision of an organ, bleeding, or the like in a SLAM image (or an observation image) may be used as reliability evaluation information.

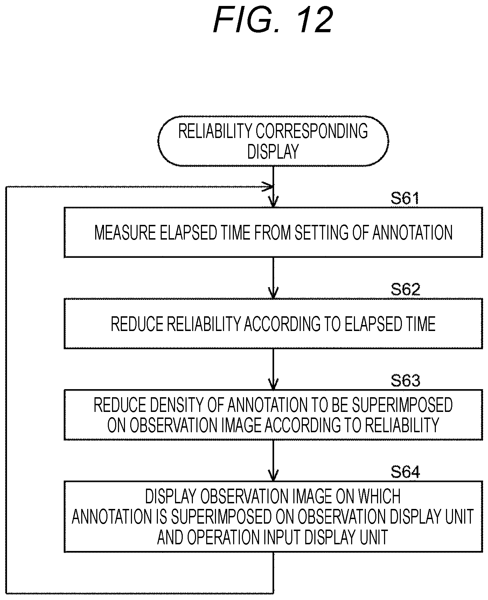

Fig. 12 is a flowchart illustrating a procedure example of the processing of displaying an annotation in the present specific example. Note that it is assumed that capturing of an observation image and a SLAM image by the imaging unit 11 and display of the observation image on which an annotation is superimposed on the observation display unit 22 and the operation input display unit 23 have been performed. In step S61, the reliability determination unit 21 starts measuring the elapsed time from a time point at which the annotation (marking) is set. The processing proceeds from step S61 to step S62. In step S62, the reliability determination unit 21 reduces the reliability according to the measured elapsed time. For example, the reliability is reduced from 5 to 0 every time a certain period of time elapses. The processing proceeds from step S62 to step S63.

In step S63, in a case of generating the image of the annotation (marking) to be superimposed on the observation image, the annotation information generation unit 43 of the display control unit 20 reduces the density of the image of the annotation (marking) according to the reliability evaluated in step S62. The processing proceeds from step S63 to step S64.

In step S64, the observation superimposition unit 41 and the operation input superimposition unit 42 of the display control unit 20 superimpose the image of the annotation (marking) generated in step S63 on the observation image from the development processing unit 12 and display the same on the observation display unit 22 and the operation input display unit 23, respectively.

By the above processing procedure, the form of the annotation (marking) to be superimposed on the observation image is changed according to the reliability. According to the processing of displaying an annotation (marking) in Fig. 12, the user can visually recognize the degree of reliability of the position of an annotation (marking) to be superimposed on an observation image. Specifically, the density of the image of the annotation (marking) gradually decreases with a lapse of time, and the annotation (marking) naturally disappears by blending with the observation image. The user can easily visually recognize that the annotation (marking) is becoming faint, and can easily recognize that the reliability of the position of the annotation (marking) is low.

### <Example of processing of calculating two-dimensional position of annotation according to reliability>

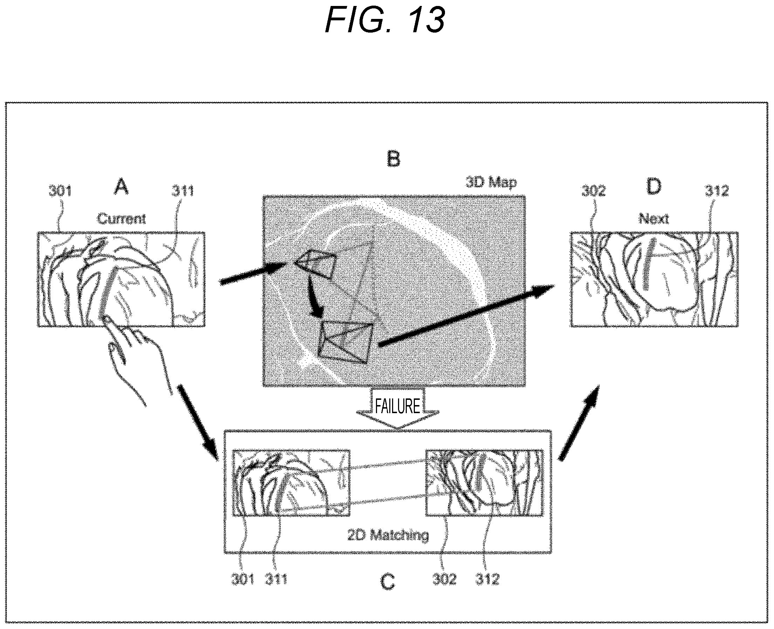

Fig. 13 is a diagram illustrating a flow of processing of calculating the two-dimensional position of an annotation performed by the annotation two-dimensional position calculation unit 19. In A of Fig. 13, it is assumed that the user designates the position of a marking 311 as an annotation on an observation image 301 displayed on the operation input display unit 23 similarly to A of Fig. 5. At this time, as illustrated in the flow from A to B in Fig. 13, the annotation three-dimensional position calculation unit 17 in Fig. 2 calculates the position on a three-dimensional map corresponding to the two-dimensional position of the annotation and stores the position in the annotation information storage unit 18. In a case where the annotation stored in the annotation information storage unit 18 is superimposed on the observation image and displayed on the observation display unit 22 and the operation input display unit 23, the annotation two-dimensional position calculation unit 19 changes the processing content for calculating the two-dimensional position of the annotation according to the reliability.

The reliability determination unit 21 evaluates the reliability on the basis of the fourth reliability evaluation information and calculates a level value of the reliability. Specifically, the reliability determination unit 21 detects the presence or absence of an estimation result of the position/posture of the imaging unit 11 by the self-position estimation unit 32 (whether or not estimation has been performed), and sets the reliability to 1 in a case where there is an estimation result, and sets the reliability to 0 in a case where there is no estimation result. In a case where the reliability is 1, the annotation two-dimensional position calculation unit 19 calculates the two-dimensional position of an annotation to be superimposed on an observation image on the basis of a three-dimensional map stored in the three-dimensional map storage unit 15, the three-dimensional position of the annotation stored in the annotation information storage unit 18, and the position/posture of the imaging unit 11 estimated by the self-position estimation unit 32 of the three-dimensional information generation unit 13, as illustrated in the flow from B to D in Fig. 13. As a result, an image for observation 302 on which a marking 312 as the annotation is superimposed is displayed on the observation display unit 22 as illustrated in D of Fig. 13.

On the other hand, in a case where the reliability is 0, the annotation two-dimensional position calculation unit 19 calculates the two-dimensional position of the annotation using information (annotation feature point information) such as feature points on a SLAM image as illustrated in the flow from C to D in Fig. 13. That is, the annotation information storage unit 18 stores, as annotation feature point information as a part of annotation information, information of feature points on a SLAM image (feature point information including the two-dimensional positions of the feature points, the feature amounts, and the like) detected by the feature point detection unit 31 of the three-dimensional information generation unit 13 at the time of setting the annotation and information indicating positional relationships between the two-dimensional position of the annotation and the feature points (positional relationship information). However, the annotation information storage unit 18 may store annotation feature point information detected in, instead of a case where the annotation designation information is set in the annotation two-dimensional position designation unit 16 in Fig. 4, a case where the self-position estimation unit 32 of the three-dimensional information generation unit 13 normally estimates the position/posture of the imaging unit 11.

In a case where the two-dimensional position of the annotation is calculated using the annotation feature point information, the annotation two-dimensional position calculation unit 19 acquires, from the feature point detection unit 31 of the three-dimensional information generation unit 13, information of feature points on a SLAM image newly captured by the imaging unit 11, and performs matching with the feature points indicated by the annotation feature point information (feature point information). As a result, the two-dimensional positions of the feature points indicated by the annotation feature point information (feature point information) are identified for the newly captured SLAM image. The annotation two-dimensional position calculation unit 19 calculates the two-dimensional position of the annotation on the newly captured SLAM image (observation image) on the basis of the identified two-dimensional positions of the feature points indicated by the annotation feature point information (feature point information) and the positional relationship information of the annotation feature point information. That is, the annotation two-dimensional position calculation unit 19 detects a movement amount by tracking (two-dimensional tracking) the feature points on the SLAM image, and calculates the two-dimensional position to which the annotation is moved on the SLAM image by the detected movement amount. As a result, the image for observation 302 on which the marking 312 as the annotation is superimposed is displayed on the observation display unit 22 as illustrated in D of Fig. 13.

According to this, even in a situation where self-position estimation cannot be performed and the two-dimensional position of the annotation cannot be appropriately calculated from the three-dimensional position of the annotation, the two-dimensional position of the annotation can be appropriately calculated from information such as the feature points on the SLAM image, and the annotation can be superimposed and displayed on the observation image. Therefore, display of the annotation on the observation image is continued seamlessly without causing the user to feel uncomfortable.

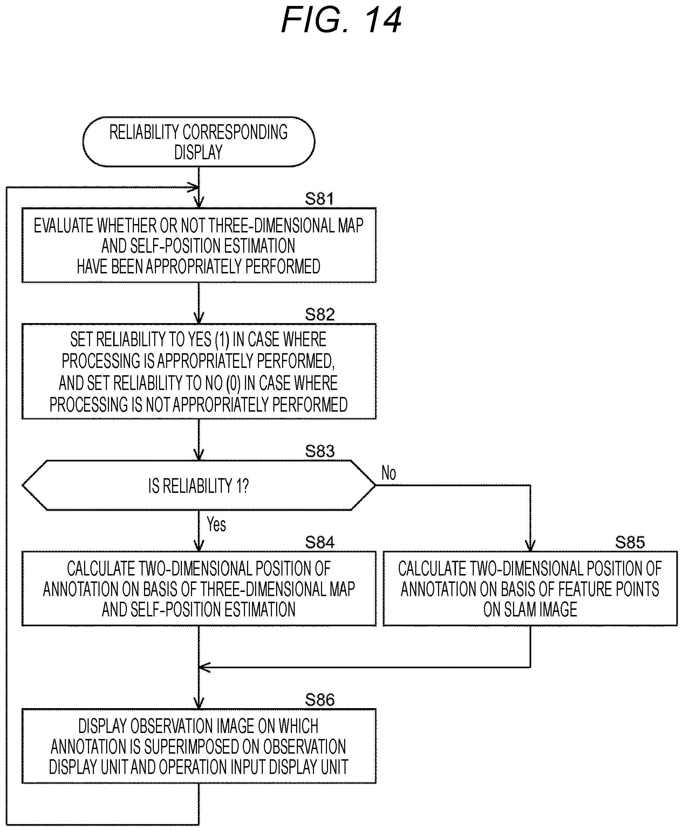

Fig. 14 is a flowchart illustrating a procedure example of the processing of calculating the two-dimensional position of an annotation performed by the annotation two-dimensional position calculation unit 19. Note that it is assumed that capturing of an observation image and a SLAM image by the imaging unit 11 and display of the observation image on which an annotation is superimposed on the observation display unit 22 and the operation input display unit 23 have been performed. In step S81, the reliability determination unit 21, the three-dimensional information generation unit 13 detects whether or not self-position estimation by the self-position estimation unit 32 has been appropriately performed. The processing proceeds from step S81 to step S82. In step S82, the reliability determination unit 21 sets the reliability to 1 in a case where the processing is appropriately performed, and sets the reliability to 0 in a case where the processing is not appropriately performed. The processing proceeds to step S83. In step S83, the annotation two-dimensional position calculation unit 19 determines whether or not the reliability is 1.

In a case where the determination is affirmative in step S83, the processing proceeds to step S84, and in a case where the determination is negative, the processing proceeds to step S85. In step S84, the annotation two-dimensional position calculation unit 19 calculates the two-dimensional position of the annotation on the basis of a three-dimensional map and the self-position estimation (position/posture of the imaging unit 11). The processing proceeds from step S84 to step S86. In step S85, the two-dimensional position of the annotation is calculated on the basis of the feature points on the SLAM image. The processing proceeds from step S85 to step S86.

In step S86, the observation superimposition unit 41 and the operation input superimposition unit 42 of the display control unit 20 superimpose the image of the annotation on the two-dimensional position of the annotation calculated in step S84 or S85 on the observation image from the development processing unit 12 and display the same on the observation display unit 22 and the operation input display unit 23, respectively. Furthermore, in a case where the image of the annotation is superimposed and displayed on the two-dimensional position of the annotation calculated in step S85, that is, in a case where the feature amount that is a determination result of the reliability determination unit 21 is 0, the annotation information generation unit 43 of the display control unit 20 notifies the user that the superimposition display of the annotation of the observation image is performed by tracking (two-dimensional tracking) of the feature points on the SLAM image without using self-position estimation (three-dimensional tracking) by changing the form of the annotation to be superimposed on the observation image or adding an annotation such as a character image. This notification may be performed by a device outside the device in a physical manner (light, vibration, or the like).

According to the above processing, even in a situation where self-position estimation cannot be performed and the two-dimensional position of the annotation cannot be appropriately calculated from the three-dimensional position of the annotation, the two-dimensional position of the annotation is appropriately calculated from information such as the feature points on the SLAM image. The annotation is superimposed and displayed on the observation image. Therefore, superimposition display of the annotation on the observation image is continued seamlessly without causing the user to feel uncomfortable.

### <Hardware configuration>

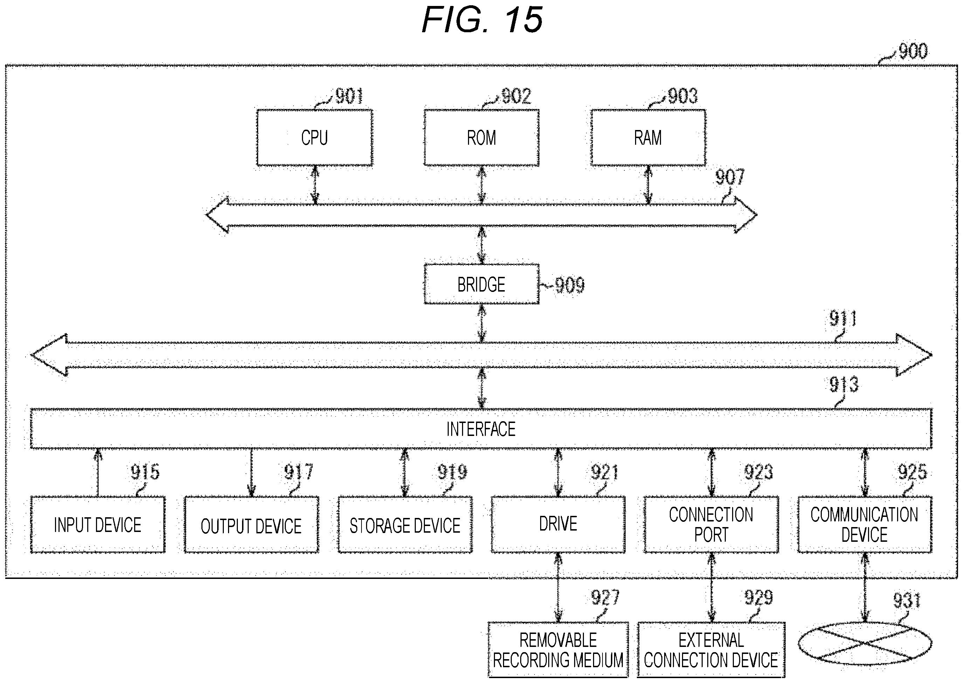

Next, an example of a hardware configuration of an information processing device included in the medical information processing system according to the embodiment of the present technology will be described in detail with reference to Fig. 15.

Fig. 15 is a block diagram illustrating an example of a hardware configuration of an information processing device 900 included in the medical information processing system according to the embodiment of the present technology.

As illustrated in Fig. 15, the information processing device 900 includes a CPU 901, a ROM 903, and a RAM 905. Moreover, the information processing device 900 includes a host bus 907, a bridge 909, an external bus 911, an interface 913, an input device 915, an output device 917, and a storage device 919. Note that the information processing device 900 may include a drive 921, a connection port 923, and a communication device 925.

The CPU 901 functions as an arithmetic processor and a control device, and controls overall operation in the information processing device 900 or a part thereof, in accordance with various programs recorded in the ROM 903, the RAM 905, the storage device 919, or a removable recording medium 927.

The ROM 903 stores programs, calculation parameters, and the like used by the CPU 901. The RAM 905 primarily stores a program used by the CPU 901, parameters that appropriately change in execution of the program, and the like. These are interconnected by the host bus 907 formed by an internal bus such as a CPU bus. Note that each configuration of an information processing unit 71 described with reference to Fig. 3 and the like is implemented by, for example, the CPU 901.

The host bus 907 is connected to the external bus 911 such as a peripheral component interconnect/interface (PCI) bus via the bridge 909. The input device 915, the output device 917, the storage device 919, the drive 921, the connection port 923, and the communication device 925 are connected to the external bus 911 via the interface 913.

The input device 915 is an operation means operated by the user, such as, for example, a mouse, a keyboard, a touch panel, a button, a switch, a lever, and a pedal. Furthermore, the input device 915 may be, for example, a remote control means (so-called remote controller) using infrared light or other radio waves. The input device 915 may be, for example, an external connection device 929 such as a mobile phone, a smartphone, or a tablet terminal corresponding to the operation of the information processing device 900.

The input device 915 includes, for example, an input control circuit or the like that generates an input signal on the basis of information input by the user using the operation means described above and outputs the input signal to the CPU 901.

By operating the input device 915, the user can input various types of data and give an instruction to perform a processing operation to the information processing device 900.

The output device 917 includes a device that can visually or audibly notify the user of acquired information. Specifically, the output device 917 is configured as a display device such as a CRT display device, a liquid crystal display device, a plasma display device, an EL display device, and a lamp, an audio output device such as a speaker and a headphone, a printer device, or the like.

The output device 917 outputs, for example, results obtained by various types of processing performed by the information processing device 900. Specifically, the display device displays results obtained by various types of processing performed by the information processing device 900 as text or images. On the other hand, the audio output device converts an audio signal including reproduced voice data, sound data, or the like into an analog signal and outputs the analog signal.

The storage device 919 is a data storage device configured as an example of a storage unit of the information processing device 900. The storage device 919 includes, for example, a magnetic storage unit device such as a hard disk drive (HDD), a semiconductor storage device, an optical storage device, or a magneto-optical storage device. The storage device 919 stores programs executed by the CPU 901 and various types of data, and the like.

The drive 921 is a reader/writer for a recording medium, and is built in or externally attached to the information processing device 900. The drive 921 reads out information recorded in the removable recording medium 927 mounted, such as a magnetic disk, an optical disk, a magneto-optical disk, or a semiconductor memory, and outputs the information to the RAM 905. Furthermore, the drive 921 can also write a record in the removable recording medium 927 mounted, such as a magnetic disk, an optical disk, a magneto-optical disk, or a semiconductor memory.

The removable recording medium 927 is, for example, a DVD medium, an HD-DVD medium, a Blu-ray (registered trademark) medium, or the like. Furthermore, the removable recording medium 927 may be a CompactFlash (registered trademark) (CF), a flash memory, a secure digital (SD) memory card, or the like. Moreover, the removable recording medium 927 may be, for example, an integrated circuit (IC) card on which a non-contact type IC chip is mounted, an electronic device, or the like.

The connection port 923 is a port for directly connecting the external connection device 929 to the information processing device 900. Examples of the connection port 923 include a universal serial bus (USB) port, an IEEE 1394 port, and a small computer system interface (SCSI) port, and the like. Other examples of the connection port 923 include an RS-232C port, an optical audio terminal, a high-definition multimedia interface (HDMI) (registered trademark) port, and the like. By connecting the external connection device 929 to the connection port 923, the information processing device 900 directly acquires various types of data from the external connection device 929 or provides various types of data to the external connection device 929.

The communication device 925 is, for example, a communication interface including a communication device or the like for connecting to a communication network (network) 931. The communication device 925 is, for example, a communication card for a wired or wireless local area network (LAN), Bluetooth (registered trademark), wireless USB (WUSB), or the like. Furthermore, the communication device 925 may be a router for optical communication, a router for asymmetric digital subscriber line (ADSL), a modem for various types of communication, or the like.

For example, the communication device 925 can transmit and receive signals to and from the Internet and other communication devices according to a predetermined protocol such as TCP/IP. Furthermore, the communication network 931 connected to the communication device 925 may be configured by a network or the like connected in a wired or wireless manner. The communication network 931 may be, for example, the Internet, a home LAN, or a communication network in which infrared communication, radio wave communication, or satellite communication is performed.

Each component of the information processing device 900 in Fig. 15 may be configured using a general-purpose member, or may be configured with hardware specialized for the function of each component. Therefore, it is possible to appropriately change the hardware configuration to be used according to the technical level at the time of implementing the embodiment of the present technology.

Moreover, a computer program for implementing each function of the information processing device 900 included in the medical information processing system according to the embodiment of the present technology can be created and mounted on a personal computer or the like. Furthermore, a computer-readable recording medium in which such a computer program is stored can be provided. The recording medium is, for example, a magnetic disk, an optical disk, a magneto-optical disk, a flash memory, or the like. Furthermore, the computer program may be distributed via, for example, a network without using a recording medium.

Note that the program executed by the computer may be a program in which processing is performed in time series in the order described in the present description, or may be a program in which processing is performed in parallel or at necessary timing such as when a call is made.

### <Others>

In the present description, a system means a set of a plurality of components (devices, modules (parts), and the like), and it does not matter whether or not all the components are in the same housing. Therefore, a plurality of devices housed in separate housings and connected to each other via a network and one device in which a plurality of modules is housed in one housing are both systems.

Note that the effects described in the present description are merely examples and are not limited, and other effects may be provided.

### <Combination examples of configurations>

Note that the present technology can also have the following configurations.
(1) A medical information processing system including:
   a three-dimensional information generation unit that generates three-dimensional information including a three-dimensional map of an operative field of a patient;
   a reliability determination unit that determines reliability of the three-dimensional information; and
   a presentation control unit that changes a presentation form of additional information provided using the three-dimensional information on a basis of reliability determined by the reliability determination unit.
(2) The medical information processing system according to (1) described above,
   in which the additional information is information indicating a three-dimensional position on the three-dimensional map.
(3) The medical information processing system according to (2) described above,
   in which the additional information is information superimposed on a captured image obtained by capturing the operative field.
(4) The medical information processing system according to any one of (1) to (3) described above,
   in which the reliability determination unit determines the reliability by a stepwise value.
(5) The medical information processing system according to (3) described above,
   in which the additional information is an additional image superimposed on a position on the captured image corresponding to the three-dimensional position.
(6) The medical information processing system according to (5) described above,
   in which the presentation control unit changes a form of the additional image as a presentation form of the additional information.
(7) The medical information processing system according to (6) described above,
   in which the presentation control unit changes a density, a color, a thickness, or a presentation state of the additional image as a form of the additional image.
(8) The medical information processing system according to (6) or (7) described above,
   in which the reliability determination unit determines the reliability on a basis of an elapsed time from setting of the captured image, and
   the presentation control unit changes a form of the additional image on a basis of the reliability.
(9) The medical information processing system according to any one of (3) to (8) described above, further including
   a two-dimensional position calculation unit that calculates a position on the captured image on which the additional information is superimposed,
   in which the two-dimensional position calculation unit changes processing of calculating a position on the captured image on which the additional information is superimposed according to the reliability.
(10) The medical information processing system according to (9) described above,
   in which the two-dimensional position calculation unit performs a change to one of first processing of calculating the position on the captured image on which the additional information is superimposed using the three-dimensional map and second processing of calculating the position on the captured image on which the additional information is superimposed without using the three-dimensional map.
(11) The medical information processing system according to (10) described above,
   in which the presentation control unit presents a fact that the position on the captured image on which the additional information is superimposed is calculated by the second processing in a case where the reliability determination unit determines that the reliability is the reliability in a case of a situation in which a change to the second processing is performed.
(12) The medical information processing system according to any one of (1) to (11) described above, further including
   an image accumulation unit that stores a captured image for a certain period obtained by capturing the operative field,
   in which the three-dimensional information generation unit generates the three-dimensional map using the captured image stored in the image accumulation unit in a case where data of the three-dimensional map is insufficient at a time at which the additional information is set.
(13) The medical information processing system according to (12) described above,
   in which the reliability determination unit determines a status of data of the three-dimensional map as the reliability, and
   the presentation control unit presents a fact that the additional information is presented using the three-dimensional map generated using the captured image stored in the image accumulation unit in a case where the reliability determination unit determines that the reliability is the reliability in a case where data of the three-dimensional map is insufficient.
(14) A medical information processing method of a medical information processing system including:
   a three-dimensional information generation unit;
   a reliability determination unit; and
   a presentation control unit,
   in which the three-dimensional information generation unit generates three-dimensional information including a three-dimensional map of an operative field of a patient,
   the reliability determination unit determines reliability of the three-dimensional information, and
   the presentation control unit changes a presentation form of additional information provided using the three-dimensional information on a basis of the reliability.
(15) A program that causes a computer to function as:
   a three-dimensional information generation unit that generates three-dimensional information including a three-dimensional map of an operative field of a patient;
   a reliability determination unit that determines reliability of the three-dimensional information; and
   a presentation control unit that changes a presentation form of additional information provided using the three-dimensional information on a basis of reliability determined by the reliability determination unit.

Note that, the present embodiment is not limited to the embodiment described above, and various modifications can be made without departing from the gist of the present disclosure. Furthermore, the effects described in the present description are merely examples and are not limited, and other effects may be provided.

### REFERENCE SIGNS LIST

1 Medical information processing system
11 Imaging unit
12 Development processing unit
13 Three-dimensional information generation unit
14 Image accumulation unit
15 Three-dimensional map storage unit
16 Annotation two-dimensional position designation unit
17 Annotation three-dimensional position calculation unit
18 Annotation information storage unit
19 Annotation two-dimensional position calculation unit
20 Display control unit
21 Reliability determination unit
22 Observation display unit
23 Operation input display unit
31 Feature point detection unit
32 Self-position estimation unit
33 Three-dimensional map generation unit
41 Observation superimposition unit
42 Operation input superimposition unit
43 Annotation information generation unit

## Claims

1. A medical information processing system comprising:
a three-dimensional information generation unit that generates three-dimensional information including a three-dimensional map of an operative field of a patient;
a reliability determination unit that determines reliability of the three-dimensional information; and
a presentation control unit that changes a presentation form of additional information provided using the three-dimensional information on a basis of reliability determined by the reliability determination unit.

2. The medical information processing system according to claim 1,
wherein the additional information is information indicating a three-dimensional position on the three-dimensional map.

3. The medical information processing system according to claim 2,
wherein the additional information is information superimposed on a captured image obtained by capturing the operative field.

4. The medical information processing system according to claim 1,
wherein the reliability determination unit determines the reliability by a stepwise value.

5. The medical information processing system according to claim 3,
wherein the additional information is an additional image superimposed on a position on the captured image corresponding to the three-dimensional position.

6. The medical information processing system according to claim 5,
wherein the presentation control unit changes a form of the additional image as a presentation form of the additional information.

7. The medical information processing system according to claim 6,
wherein the presentation control unit changes a density, a color, a thickness, or a presentation state of the additional image as a form of the additional image.

8. The medical information processing system according to claim 6,
wherein the reliability determination unit determines the reliability on a basis of an elapsed time from setting of the captured image, and
the presentation control unit changes a form of the additional image on a basis of the reliability.

9. The medical information processing system according to claim 3, further comprising
a two-dimensional position calculation unit that calculates a position on the captured image on which the additional information is superimposed,
wherein the two-dimensional position calculation unit changes processing of calculating a position on the captured image on which the additional information is superimposed according to the reliability.

10. The medical information processing system according to claim 9,
wherein the two-dimensional position calculation unit performs a change to one of first processing of calculating the position on the captured image on which the additional information is superimposed using the three-dimensional map and second processing of calculating the position on the captured image on which the additional information is superimposed without using the three-dimensional map.

11. The medical information processing system according to claim 10,
wherein the presentation control unit presents a fact that the position on the captured image on which the additional information is superimposed is calculated by the second processing in a case where the reliability determination unit determines that the reliability is the reliability in a case of a situation in which a change to the second processing is performed.

12. The medical information processing system according to claim 1, further comprising
an image accumulation unit that stores a captured image for a certain period obtained by capturing the operative field,
wherein the three-dimensional information generation unit generates the three-dimensional map using the captured image stored in the image accumulation unit in a case where data of the three-dimensional map is insufficient at a time at which the additional information is set.

13. The medical information processing system according to claim 12,
wherein the reliability determination unit determines a status of data of the three-dimensional map as the reliability, and
the presentation control unit presents a fact that the additional information is presented using the three-dimensional map generated using the captured image stored in the image accumulation unit in a case where the reliability determination unit determines that the reliability is the reliability in a case where data of the three-dimensional map is insufficient.

14. A medical information processing method of a medical information processing system including:
a three-dimensional information generation unit;
a reliability determination unit; and
a presentation control unit,
wherein the three-dimensional information generation unit generates three-dimensional information including a three-dimensional map of an operative field of a patient,
the reliability determination unit determines reliability of the three-dimensional information, and
the presentation control unit changes a presentation form of additional information provided using the three-dimensional information on a basis of the reliability.

15. A program that causes a computer to function as:
a three-dimensional information generation unit that generates three-dimensional information including a three-dimensional map of an operative field of a patient;
a reliability determination unit that determines reliability of the three-dimensional information; and
a presentation control unit that changes a presentation form of additional information provided using the three-dimensional information on a basis of reliability determined by the reliability determination unit.
